# EUROPEAN PATENT SPECIFICATION

(11) **EP 3 630 802 B1**
(45) Date of publication and mention of the grant of the patent: **20.11.2024**
(21) Application number: 18732487.6
(22) Date of filing: 24.05.2018
(51) Int. Cl.: C07K 14/435, C12N 1/06, C07K 1/14, C12N 9/42

(54) **METHOD FOR THE EXTRACTION OF RECOMBINANT PROTEINS**
VERFAHREN ZUR EXTRAKTION VON REKOMBINANTEN PROTEINEN
PROCÉDÉ D'EXTRACTION DE PROTÉINES RECOMBINANTES

(30) Priority: 25.05.2017 IT 201700056835
(43) Date of publication of application: 08.04.2020
(73) Proprietor: Mascia Brunelli S.p.A., 20128 Milano (MI) (IT)
(72) Inventor: HOCHKOEPPLER, Alejandro, 40129 Bologna (BO) (IT); SCALZO, Alessandro, 73100 Lecce (LE) (IT)
(74) Representative: Ripamonti, Enrico
(86) International application number: PCT/IB2018/053674
(87) International publication number: WO 2018/215959

(56) References cited:
- WO-A2-02/26966
- US-A1- 2014 205 693
- DAKHOVA O N ET AL: "Cloning and Expression in Escherichia coli of Thermotoga neapolitana Genes Coding for Enzymes of Carbohydrate Substrate Degradation", BIOCHEMICAL AND BIOPHYSICAL RESEARCH COMMUNICATIONS, ELSEVIER, AMSTERDAM, NL, vol. 194, no. 3, 16 August 1993 (1993-08-16), pages 1359 - 1364, XP024767100, ISSN: 0006-291X, [retrieved on 19930816], DOI: 10.1006/BBRC.1993.1974
- BOK J-D ET AL: "Purification, characterization, and molecular analysis of thermostable cellulases CelA and CelB from Thermotoga neapolitana", APPLIED AND ENVIRONMENTAL MICROBIOLOGY,, vol. 64, no. 12, 1 December 1998 (1998-12-01), pages 4774 - 4781, XP002330149, ISSN: 0099-2240
- VOORHORST W G B ET AL: "CHARACTERIZATION OF THE CELB GENE CODING FOR BETA-GLUCOSIDASE FROM THE HYPERTHERMOPHILIC ARCHAEON PYROCOCCUS FURIOSUS AND ITS EXPRESSION AND SITE-DIRECTED MUTATION IN ESCHERICHIA COLI", JOURNAL OF BACTERIOLOGY, AMERICAN SOCIETY FOR MICROBIOLOGY, US, vol. 177, no. 24, 1 December 1995 (1995-12-01), pages 7105 - 7111, XP002064570, ISSN: 0021-9193
- "Current Protocols in Protein Science", 3 November 2014, JOHN WILEY & SONS, INC., Hoboken, NJ, USA, ISBN: 978-0-471-14086-3, article PAUL T. WINGFIELD: "Preparation of Soluble Proteins from Escherichia coli : Preparation of Soluble Proteins from Escherichia coli", pages: 6.2.1 - 6.2.22, XP055438649, DOI: 10.1002/0471140864.ps0602s78
- REASMEY TAN ET AL: "Effect of heat-alkaline treatment as a pretreatment method on volatile fatty acid production and protein degradation in excess sludge, pure proteins and pure cultures", BIORESOURCE TECHNOLOGY,, vol. 118, 11 May 2012 (2012-05-11), pages 390 - 398, XP028504882, ISSN: 0960-8524, [retrieved on 20120522], DOI: 10.1016/J.BIORTECH.2012.05.064
- HEIKER J T ET AL: "Access to gram scale amounts of functional globular adiponectin from E. coli inclusion bodies by alkaline-shock solubilization", BIOCHEMICAL AND BIOPHYSICAL RESEARCH COMMUNICATIONS, ELSEVIER, AMSTERDAM, NL, vol. 398, no. 1, 16 July 2010 (2010-07-16), pages 32 - 37, XP027298188, ISSN: 0006-291X, [retrieved on 20100610], DOI: 10.1016/J.BBRC.2010.06.020
- MAYANK SARASWAT ET AL: "Preparative Purification of Recombinant Proteins: Current Status and Future Trends", BIOMED RESEARCH INTERNATIONAL, vol. 2013, 1 January 2013 (2013-01-01), pages 1 - 18, XP055429379, ISSN: 2314-6133, DOI: 10.1155/2013/312709

## Description

### TECHNICAL BACKGROUND OF THE INVENTION

The present invention is directed to a method for extracting recombinant proteins from bacteria, by means of pH shock.

### STATE OF THE ART

Recombinant proteins can be produced in large amounts by means of recombinant DNA technology, which provides for the transfer into a host cell of a circular DNA molecule (called plasmid or expression vector), comprising a polynucleotide encoding for a protein of interest, heterologous to said host cell. Said polynucleotide is under the control of regulatory sequences recognised by the host cell; the latter, grown in controlled conditions, thus expresses the protein of interest¹⁰.

Recombinant peptides, proteins and enzymes are widely used for therapeutic, research or industrial purposes.

For example, recombinant cellulases are used for various industrial technologies⁹ such as the production of paper and derivatives, or of bioethanol, and for an effective conversion of cellulosic biomass into glucose.

The host cells most widely used for producing recombinant proteins are microorganisms, in particular bacteria or yeasts. Yeasts are able to secrete in the culture medium the produced proteins, facilitating their recovery (step "downstream" of the recombinant proteins production process). Bacteria, on the contrary, store the produced proteins in their cytosol or periplasm, making it necessary to use methods to extract these latter from the biomass. However, the use of bacteria as host cells is very advantageous and economical, as bacteria can be easily and rapidly grown and produce high yields, at times greater than one gram of recombinant protein per litre of microbial culture.

The extraction processes used today to recover recombinant proteins from bacterial biomass (for example mechanical, such as extraction using presses or lysis of the biomass by silica microfragments, or chemical processes, such as extraction using detergents) require considerable energy inputs, provide low yields or can even provide degraded and biologically inactive proteins.

US4464295A describes extracting recombinant proteins from gram-negative bacteria using a solution of sodium dodecyl sulphate (SDS).

The documents:
- DAKHOVA O N ET AL :"Cloning and Expression in Escherichia coli of Thermotoga neapolitana Genes Coding for Enzymes of Carbohydrate Substrate Degradation" (BIOCHEMICAL AND BIOPHYSICAL RESEARCH COMMUNICATION, ELSEVIER; AMSTERDAM, NL, vol. 194, no. 3, 16 August 1993);
- BOK J-D ET AL: "Purification, characterization, and molecular analysis of thermostable cellulase CelA and CelB from Thermotoga neapolitana" (APPLIED AND ENVIRONMENTAL MICROBIOLOGY, vol. 64, no. 12, 1 December 1998, pages 4774-4781);
- VOORHORST W G B ET AL:"Characterization of the CelB gene coding for beta-glucosidase from the hyperthermophilic archaeon Pyrococcus furiosus and its expression and site-directed mutation in Escherichia coli" (JOURNAL OF BACTERIOLOGY, AMERICAN SOCIETY FOR MICROBIOLOGY, US, vol. 177, no 24, 1 December 1995) disclose methods for cloning, purification, characterization and analysis of enzymes from E. coli. Paul T. Wingfield: "Preparation of Soluble Proteins from Escherichia coli" Current Protocols In Protein Science, 3 November 2014, John Wiley and Sons, Inc. Hoboken, NJ, USA, ISBN:978-0-471 - 14086-3, pages 6.2.1-6.2.22 discloses a method of isolating soluble proteins from E. coli cells.

REASMEY TAN ET AL: "Effect of heat-alkaline treatment as a pretreatment method on volatile fatty acid production and protein degradation in excess sludge, pure proteins and pure cultures" - BIORESOURCE TECHNOLOGY, vol. 118, 11 May 2012, pages 390-398 discloses the effect of prolonged exposure to alkaline pH of the bacterial cells enzymes, namely a large quantity of specific enzymes and that many other proteins are destroyed by treatment.

WO02/26966A2 discloses a method a used for protein isolation.

US2014/205693 A1 discloses the suitability of alkaline lysis of bacterial biomass after for example fermentation to yield a large amount of bacteria.

Lysis by sonication is a very effective extraction method, but cannot be used on an industrial scale, both due to the difficulty of processing large amounts of biomass and to the considerable amount of energy required.

For a wider application, which includes industrial scale, it is therefore desirable to obtain a method to extract recombinant proteins from bacteria that is less expensive and gives a high yield. Moreover, it is highly desirable for said extracted recombinant proteins to have biological activity.

### BRIEF DESCRIPTION OF THE INVENTION

In a first aspect, the present invention is directed to a method for extracting recombinant proteins from bacteria, comprising the step of:
i. Adding a basic solution to a suspension of bacteria containing said recombinant proteins, thus obtaining an incubation mixture having a pH of 12 or more;
ii. Incubating said bacteria in said incubation mixture having a pH in the rnge of 12 and 14;
iii. At the nd of the incubation, adding a volume of acid solution to the incubation mixture, such as to obtain a final mixture having a pH ranging between 7 and 9;
iv. Purifying the recombinant proteins extracted from the incubation mixture or from the final mixture;
Wherein:
- Said basic solution is a solution of sodium hydroxide (NaOH);
- Said bacteria are incubated in said incubation mixture for a time ranging between 1 and 3 hours;
- Said bacteria are incubated in said incubation mixture at a temperature ranging between 18°C and 30°C;
And wherein said bacteria are gram negative.

The method of the present invention allows recombinant proteins to be extracted economically and with high yields. Surprisingly, recombinant proteins extracted with said method maintain their biological activity.

In another aspect the invention is directed to the use of a basic solution, preferably a solution of sodium hydroxide (NaOH) for extracting recombinant proteins from bacteria.

### BRIEF DESCRIPTION OF THE FIGURES

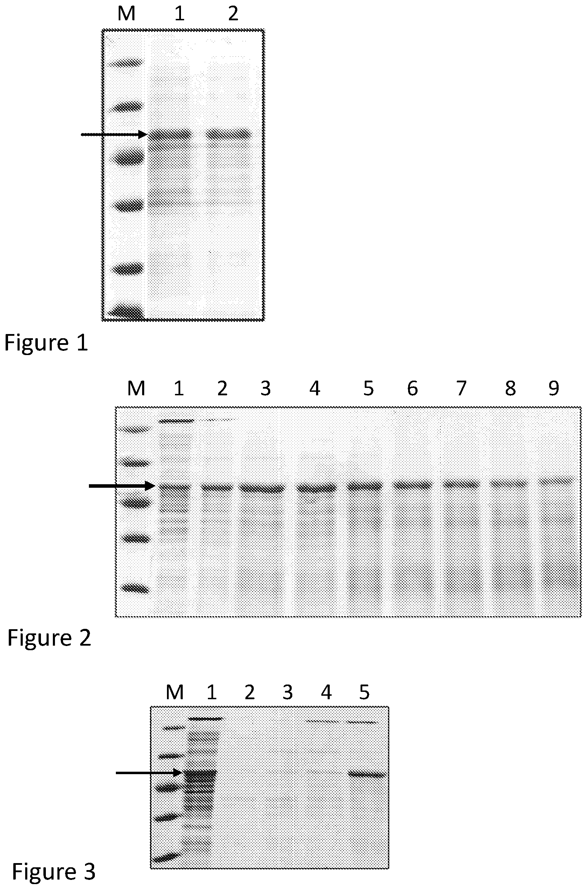

Fig. 1: SDS-PAGE analysis of the extract from a suspension of bacteria transformed to express enzyme 2, obtained by means of sonication. Lane 1=total extract; lane 2=soluble extract; M = molecular weight marker; the arrow indicates the enzyme band.
Fig. 2: SDS-PAGE analysis of the soluble fraction of cellular extracts from a suspension of bacteria transformed to express enzyme 2, obtained by means of sonication (lane 1) or by incubation at pH 13 and at 45°C at different times (lanes 2 to 9: respectively at 0, 10, 30, 60, 120, 180, 270, 360 minutes), according to a preferred embodiment of the method of the present invention; M = molecular weight marker; the arrow indicates the enzyme band.
Fig. 3: SDS-PAGE analysis of the soluble fraction of cellular extracts from a suspension of bacteria transformed to express enzyme 2, obtained by means of sonication (lane 1) or by incubation at 20°C for 15 hours at pH 10 (lane 2), 11 (lane 3), 12 (Lane 4) or 13 (lane 5); M = molecular weight marker; the arrow indicates the enzyme band.

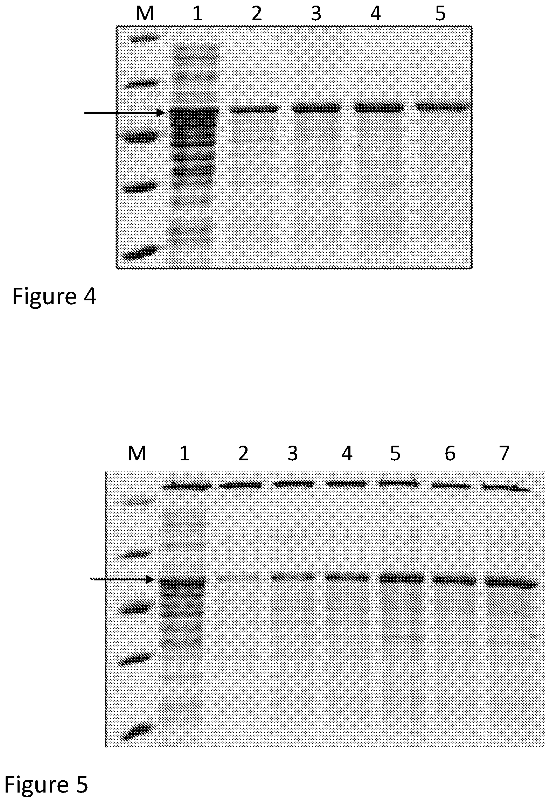

Fig. 4: SDS-PAGE analysis of soluble cellular extracts of a suspension of bacteria transformed to express enzyme 2, obtained by means of sonication (lane 1) or by incubation at pH 13 at room temperature for different times (3 hours, lane 2; 6 hours, lane 3; 9 hours, lane 4; 15 hours, lane 5); M = molecular weight marker; the arrow indicates the enzyme band.
Fig. 5: SDS-PAGE analysis of soluble cellular extracts of a suspension of bacteria transformed to express enzyme 2, obtained by means of sonication (lane 1) or by incubation at pH 13, at room temperature for 1, 2, 3, 4, 5 or 6 hours (respectively in lanes 2 to 7); M = molecular weight marker; the arrow indicates the enzyme band.

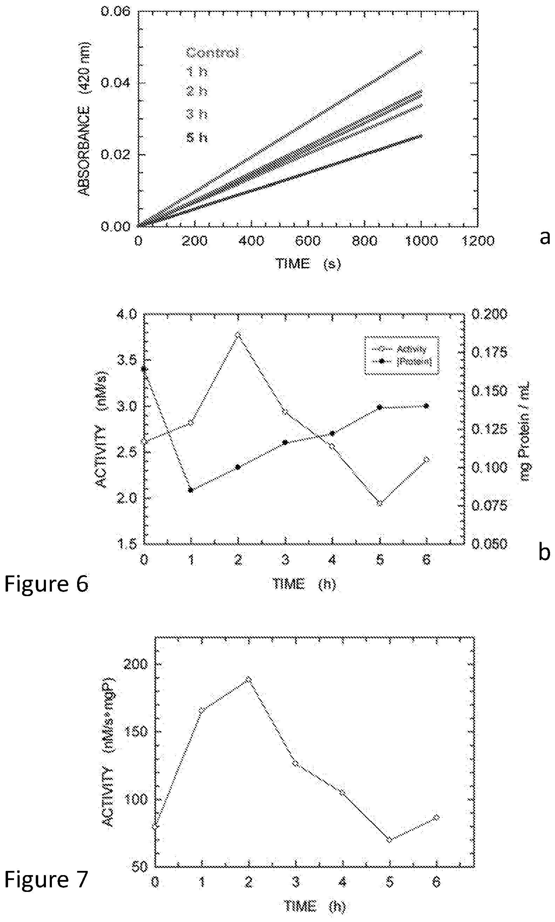

Fig. 6: Analysis of the β-glucosidase activity of extracted proteins in Fig. 5. (a) spectrophotometric measurement at different reaction times; (b) β-glucosidase activity found (nM/s), empty circles, and amount of protein per mL (mgP/mL), solid circles, present in the samples extracted at different incubation times (in the abscissa). The control, corresponding to the extract obtained by means of sonication, is shown in the ordinate.
Fig. 7: specific β-glucosidase activity (nM/s*mgP) of each sample of Fig. 5 extracted at different incubation times (in the abscissa). The control, corresponding to the extract obtained by means of sonication, is shown in the ordinate.

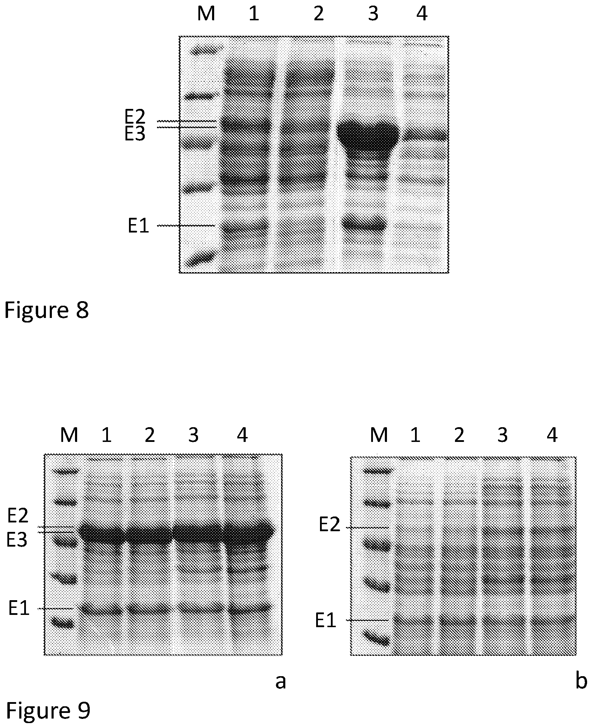

Fig. 8: SDS-PAGE analysis of total cellular extracts (lanes 1 and 3) or of the soluble fraction (lanes 2 and 4) obtained by sonication of a suspension of bacteria transformed to co-express enzymes 1 and 2 (E1 and E2) alone (lanes 1 and 2), or together with enzyme 3 (E3) (lanes 3 and 4); M=molecular weight marker.
Fig. 9: SDS-PAGE analysis of total cellular extracts or of their soluble fraction of: a) a suspension of bacteria transformed to express the enzymes 1, 2 and 3 (E1, E2, E3); b) a suspension of bacteria transformed to express the enzymes 1 and 2. The extracts are obtained by incubation at pH 13, for 2 or 2.5 hours, according to preferred embodiments of the method of the invention. In a) and b): M=molecular weight marker; lane 1=soluble extracts at 2 hours; lane 2= soluble extracts at 2.5 hours; lane 3=total extracts at 2 hours; lane 4=total extracts at 2.5 hours.

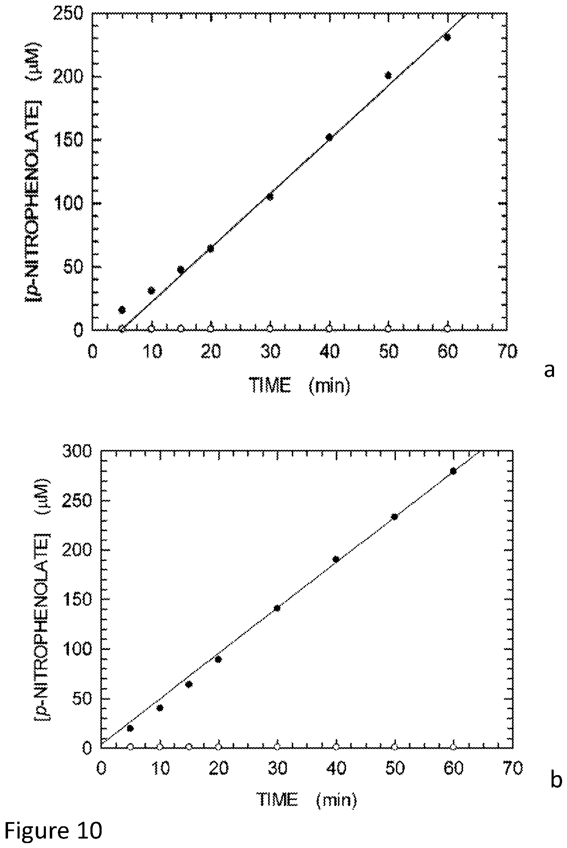

Fig. 10: Reaction kinetics of beta-glucosidase activity at 80 °C, of soluble extracts obtained by incubation at pH 13 at 25 °C of a suspension of bacteria transformed to express enzymes 1 and 2 (Fig. 10 a) or enzymes 1, 2 and 3 (Fig. 10 b). In each graph the control sample (white) is represented by white circles.

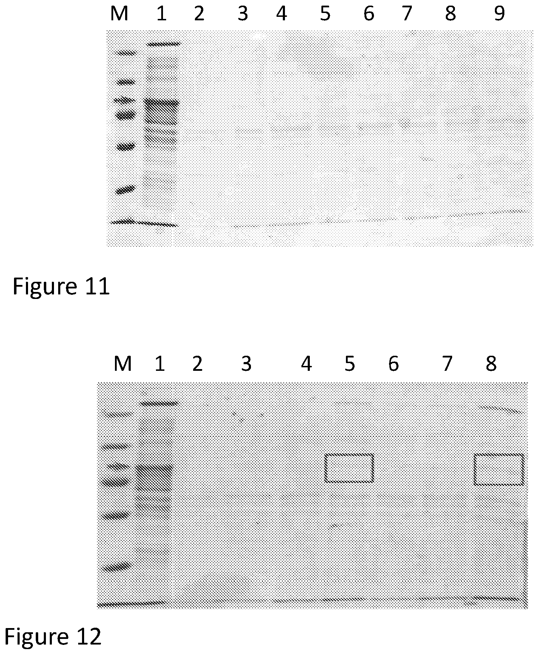

Fig. 11: SDS-PAGE analysis of cellular extracts of a suspension of bacteria transformed to express enzyme 2, obtained by incubation at pH 9 at 45°C for 0, 10, 30, 60, 120, 180, 270 and 360 minutes of incubation (lanes 2-9, respectively), compared with the soluble protein extract obtained with sonicator (lane 1); M=molecular weight marker; the arrow indicates the enzyme band.
Fig. 12: SDS-PAGE analysis of cellular extracts of a suspension of bacteria transformed to express enzyme 2, obtained by incubation at pH 10, 11 or 12, at 56°C. They are loaded in the following order in lanes 2 to 8: soluble protein extract obtained with sonicator, control sample (without NaOH) incubated for 10 minutes, extracts at pH 10 for 10 minutes, at pH 11 for 10 minutes, at pH 12 for 10 minutes, at pH 10 for 1 hour, at pH 11 for 1 hour, at pH 12 for 1 hour) ; M=molecular weight marker; the arrow indicates the enzyme band.

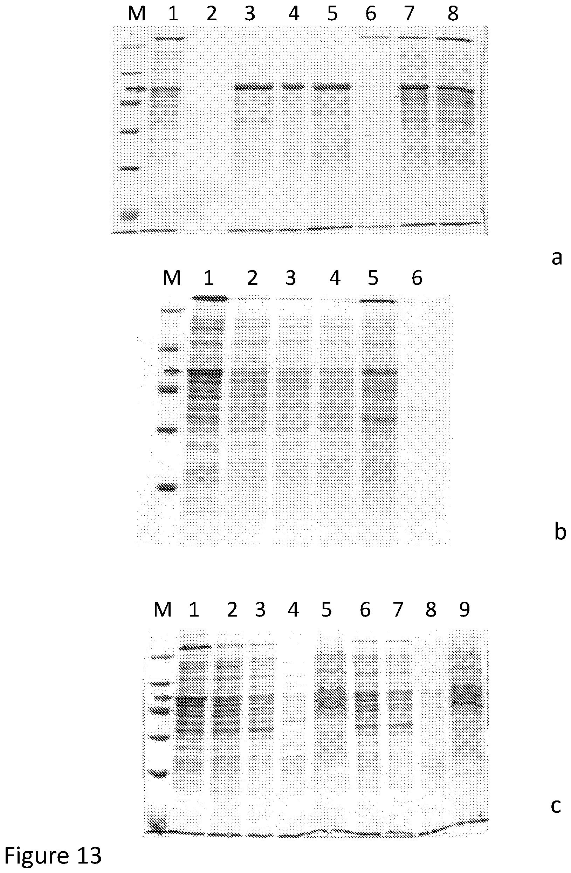

Fig. 13: SDS-PAGE analysis of cellular extracts of a suspension of bacteria transformed to express enzyme 2, obtained by extraction with detergents: a) they are loaded in the following order in lanes 1 to 8: soluble protein extract obtained with sonication, control sample (without NaOH) incubated for 1 hour, extracts in basic solution at pH 13 for 10 minutes, at pH 13 for 30 minutes, at pH 13 for 1 hour, at pH 12 for 1 hour, at pH 12 plus SDS 1% for 1 hour, at pH 12 plus SDS 0.1% for 1 hour; M=molecular weight marker; the arrow indicates the enzyme band; b) they are loaded in the following order in lanes 1 to 6: soluble protein extract obtained with sonication, extract in basic solution at pH 9 plus SDS 0.1% for 1 hour, at pH 10 plus SDS 0.1% for 1 hour, at pH 11 plus SDS 0.1% for 1 hour, at pH 12 plus SDS 0.1% for 1 hour, control sample (without NaOH) for 1 hour; M=molecular weight marker; the arrow indicates the enzyme band; c) they are loaded in the following order in lanes 1 to 9: soluble protein extract obtained with sonicator, extract at pH 8 plus SDS 0.1%, at pH 8 plus SDS 1%, at pH 8 plus CTAB 0.1%, at pH 8 plus CTAB 1%, at pH 9 plus SDS 0.1%, at pH 9 plus SDS 10, at pH 9 plus CTAB 0.1%, at pH 9 plus CTAB 10; M=molecular weight marker; the arrow indicates the enzyme band.

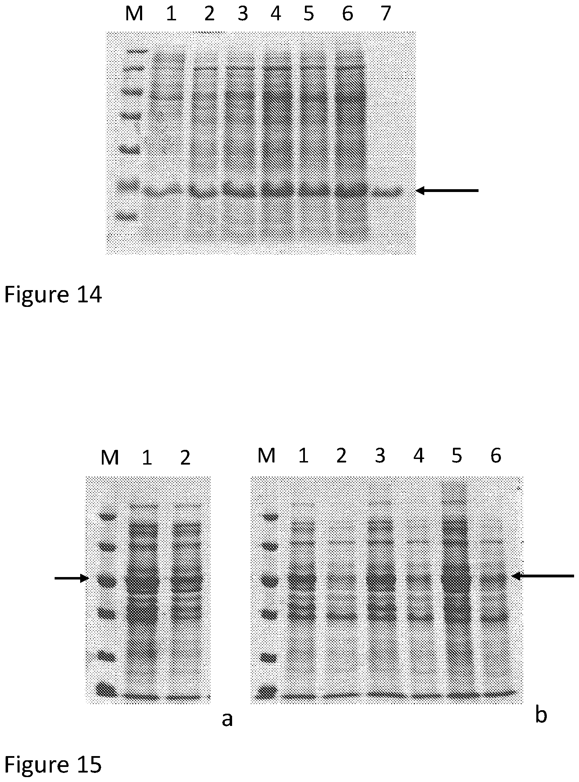

Fig. 14: SDS-PAGE analysis of soluble cellular extracts of a suspension of bacteria transformed to express tyrosine phosphatase of *Mycobacterium tuberculosis.* The extract obtained with sonication is loaded in lane 1, the extracts obtained with extraction at pH 13 respectively for: 30 minutes, 1 hour, 2 hours, 4 hours, 6 hours are loaded in lanes 2 to 6; the purified protein (4 µg) is loaded in lane 7; M=molecular weight marker; the arrow indicates the band of the recombinant protein.
Fig. 15: SDS-PAGE analysis of cellular extracts of a suspension of bacteria transformed to express a synthetic Klenow sub-fragment (HoLaMa). In a) the total and soluble protein extracts, obtained with sonication, are loaded respectively in lane 1 and 2; in b) the total and soluble protein extracts, obtained at pH 13 for 30 minutes (lanes 1 and 2), for 1 hour (lanes 3 and 4), for 2 hours (lanes 5 and 6) are loaded respectively; the arrow indicates the band of the recombinant protein.

### DETAILED DESCRIPTION OF THE INVENTION

In a first aspect, the present invention is directed to a method for extracting recombinant proteins from bacteria, comprising the step of:
i.Adding a basic solution to a suspension of bacteria containing said recombinant proteins, thus obtaining an incubation mixture having a pH of 12 or more;
ii.Incubating said bacteria in said incubation mixture having a pH in the range of 12 and 14;
iii.At the end of the incubation, adding a volume of acid solution to the incubation mixture, such as to obtain a final mixture having a pH ranging between 7 and 9;
iv.Purifying the recombinant proteins extracted from the incubation mixture or from the final mixture;
Wherein:
- Said basic solution is a solution of sodium hydroxide (NaOH);
- Said bacteria are incubated in said incubation mixture for a time ranging between 1 and 3 hours;
- Said bacteria are incubated in said incubation mixture at a temperature ranging between 18°C and 30°C;
And wherein said bacteria are gram negative.

The method of the present invention allows recombinant proteins to be extracted economically and with high yields. Surprisingly, recombinant proteins extracted with said method maintain their biological activity.

In aother aspect the invention is directed to the use of a basic solution, preferably a solution of sodium hydroxide (NaOH) for extracting recombinant proteins from bacteria.

The term "recombinant protein" means a protein produced in a host cell, by means of recombinant DNA technology; the recombinant protein is heterologous to said host cell, that is, it is not naturally present in said host cell. Said recombinant protein can be identical, or almost, to the same protein produced in its natural organism.

Preferably, recombinant proteins extracted according to the method of the present invention are thermostable proteins. With thermostable protein it is meant a protein capable of maintaining its native structure at temperatures above 40-50 °C.

More preferably, recombinant proteins extracted according to the method of the present invention are enzymes of the cellulase family. Cellulases are a family of hydrolase enzymes, which catalyse hydrolysis of the 1,4-β-D-glycosidic bonds in the cellulose. Examples of cellulases include CelB from *Thermotoga neapolitana,* Cel6B from *Thermobifida fusca* and Ggha from *Thermotoga neapolitana* ¹⁻⁵.

Non-exhaustive examples of further recombinant proteins extracted with the method of the present invention include: human or murine interferon, such as human interferon alpha 1, alpha 8 or alpha 21 and murine interferon alpha 1, proteases, such as trypsin inhibitor from *Sinapis alba* MTI-2, CRM197, phosphatases, such as tyrosine-phosphatases from *Mycobacterium tuberculosis* (SEQ ID NO. 6), synthetic Klenow sub-fragment (HoLaMa) from *Escherichia coli* (SEQ ID NO.7) and others.

Preferably, the bacteria that contain said recombinant proteins are transformed with one or more expression vectors, in which at least one polynucleotide encoding for a recombinant protein is cloned. Preferably, said at least one polynucleotide encodes for an enzyme of the cellulase family. Preferably, said at least one polynucleotide is a polynucleotide of sequence selected from the sequences SEQ ID No. 1, SEQ ID No. 2 or SEQ ID No. 3, or of sequence having at least 70%, at least 80%, at least 90% or at least 950 of identity with a sequence selected from the sequences SEQ ID No. 1, SEQ ID No. 2 or SEQ ID No. 3.

In a preferred aspect, the transformed bacteria contain at least two different recombinant proteins, preferably at least two different cellulases, in each bacterial cell.

Preferably, in the method of the present invention the recombinant proteins are extracted from a suspension of bacteria: said suspension of bacteria preferably consists of a pellet of bacteria, for example obtained by centrifugation of a bacterial culture in liquid medium, resuspended in a buffer, for example in a phosphate buffer, having a pH ranging between 7 and 9, preferably approximately equal to 8. Preferably, the molarity of the buffer is less than 100mM.

Preferably, the method for extracting recombinant proteins from bacteria according to the present invention comprises in succession: adding a basic solution to a suspension of bacteria containing said recombinant proteins, obtaining an incubation mixture having a pH greater than or equal to 12; incubating said bacteria in said incubation mixture having a pH greater than or equal to 12.

Preferably, the incubation time of the suspension of bacteria in the incubation mixture has a duration of 2 hours, even more preferably approximately 1 hour.

In a particularly preferred embodiment of the present invention, a suspension of bacteria is incubated in an incubation mixture having a pH of 13, for a time ranging between 1 and 3 hours, preferably for a time of approximately 2 hours, at room temperature.

The person skilled in the art is in any case able to establish optimal conditions of incubation time for each extraction reaction. For example, heat-sensitive proteins, that is, proteins that denature at temperatures of above 40-50°C, are preferably extracted with the method of the present invention by incubating the suspension of bacteria for a time ranging between 10 minutes and 3 hours, more preferably from 30 minutes to 2 hours, even more preferably for a time below 2 hours.

Preferably, incubation takes place maintaining the incubation mixture under stirring, for example with the aid of a magnet and a magnetic stirrer.

Preferably incubation takes place at room temperature. Room temperature means a temperature of approximately 20 °C.

At the end of incubation, a mixture comprising the extracted recombinant proteins is obtained.

Preferably, the purification is carried out by flocculation or centrifugation of the mixture comprising the extracted recombinant proteins and recovery of the supernatant containing the extracted recombinant proteins (soluble fraction).

Preferably, the recombinant proteins extracted by the method of the present invention are biologically active. A protein is biologically active when it is able to produce a biological effect in a biological organism and/or on a biological material. For example, an enzyme is a biologically active protein when it is able to catalyse a biological reaction.

The basic solution utilised in the method of the present invention is a solution of a strong base: sodium hydroxide (NaOH), preferably having a concentration of approximately 0.2M; it must be noted that this solution is highly economical.

The step of terminating incubation by adding an acid solution to the incubation mixture, in preferably in a a volume such as to obtain a mixture having a pH approximately equal to 8. Preferably, said acid solution is a solution of a strong acid; examples of strong acids that can be utilised in the method of the present invention include: hydrochloric acid (HCl), hydrofluoric acid (HF), formic acid (HCOOH), acetic acid (CH₃COOH), citric acid and other carboxylic acids. More preferably, this acid solution is a solution of hydrochloric acid (HCl), more preferably having a concentration of approximately 0.2 M. It must be noted that this solution is highly economical.

The volume of basic solution or of acid solution to be added to the bacterial suspension or to the incubation mixture to obtain the desired pH is determined with methods known in the art. For example: to obtain an incubation mixture having a pH value of 13, the same volume of NaOH 0.2 M can be added to a bacterial cell suspension resuspended in phosphate buffer 100 mM, pH 8. After the desired time has elapsed, incubation can be terminated by adding HCl 0.2 M in the same volume as the one of NaOH 0.2 M utilised previously.

The pH of the solutions, of the mixtures and of the suspensions utilised in the present invention can be easily measured by techniques known in the art, for example by the use of a pH meter.

The bacteria employed in the method of the present invention are preferably Gram-negative bacteria, such as, for example: *Escherichia coli (E. coli)* bacteria, or bacteria of the *Salmonella, Shigella, Yersinia,* or *Klebsiella* species, or *Pseudomonas fluorescens* bacteria, or derivates. More preferably, said bacteria are of the *Escherichia coli* species, more preferably of the strain BL21(DE3) or of the strain TOP10.

Bacterial cells of these species can be easily obtained from commercial suppliers or from biological material collections, such as ATCC (https://www.lgcstandards-atcc.org) or CGSC (http://cgsc2.biology.yale.edu/index.php).

Methods for the expression of recombinant proteins in bacteria are known in the art. In particular, the bacteria utilised in the method of the present invention are preferably bacteria transformed with one or more expression vectors, according to methods known in the art, such as electroporation or thermal shock, and grown in conditions suitable for the production of recombinant proteins, for example grown in a growth medium in a flask or in a bioreactor, for a time sufficient to express the protein of interest. The bacteria utilised in the method of the present invention are thus bacteria transformed with one or more vectors for the expression of recombinant proteins and that contain the recombinant proteins produced.

Preferably, the method of the present invention does not comprise a step of mechanical extraction of the recombinant proteins, such as sonication.

Preferably, the method of the present invention does not comprise a step of extraction with detergents.

Further characteristics and advantages of the present invention will be apparent from the description of preferred, but not exclusive, embodiments of the method of the invention, exemplified hereafter.

### EXAMPLES

### EXAMPLE 1: synthesis of recombinant proteins

*Escherichia coli* bacteria of the strain BL21 (DE3), obtained from the CGSC public collection (Catalogue number: CGSC 12504)¹¹ or as commercial products from Novagen^{®} (catalogue number 69450)¹², were utilised in the working examples of the present invention. The genotype of these bacteria is: *F- ompT, gal, dcm, lon, hsdSB (rB⁻ mB⁻), λDE3 (lacI, lacUV5-T7 gene1, indl, sam7, nin5) .*

The bacteria were transformed with the plasmid pETDuet-1 (SEQ ID No. 4) obtained from Novagen^{®} (Cat. No. 71146-3) and/or with the plasmid pACYCDuet-1 obtained from Novagen^{®} (Cat. No. 71147-3) (SEQ ID No. 5), each comprising two multiple cloning sites. Each plasmid thus allows the simultaneous co-expression of two genes. Each multiple cloning site is under the control of the regulatory sequences of the phage T7^{7,6,8}, recognised by the RNA polymerase of bacteria of the strain BL21 (DE3) . In particular, a polynucleotide having SEQ ID No. 1 (hereinafter "gene 1"), encoding for an endocellulase derived from the cellulase CelB from *Thermotoga neapolitana* (Uniprot accession number: P96492_THENE, hereinafter enzyme 1, having molecular weight 29.8 kDa) was cloned in the vector pETDuet-1 utilising the restriction sites NcoI and PstI. A polynucleotide of sequence SEQ ID NO. 2 (hereinafter "gene 2"), encoding the beta-glucosidase enzyme CelB from *Pyrococcus furiosus* (Uniprot accession number: Q51723_9EURY, hereinafter "enzyme 2", having molecular weight 54.7 kDa), was cloned in the same plasmid utilising the restriction sites NdeI and KpnI. A polynucleotide having SEQ ID No. 3 ("gene 3"), encoding for the variant I170T of the cellulase Ggha from *Thermotoga neapolitana* (Uniprot accession number: P0C946, BGLA_THENE, hereinafter "enzyme 3", having molecular weight 49.4 kDa) was cloned in the vector pACYCDuet-1 utilising the restriction sites NcoI and PstI.

For transformation of *E. coli* BL21(DE3) cells obtained from Novagen^{®}, the indications provided by the manufacturer were followed. For transformation of electrocompetent *E. coli* BL21(DE3) obtained from the CGSC public collection, 5 ng of plasmid DNA were introduced by electroporation, applying a pulse of 1.8 kV at 200 Ω by GenePulsar II (Bio-Rad). The electroporated bacteria were resuspended in cooled SOC medium (LB liquid medium to which glucose 20 mM, MgCl2 10 mM and KCl 2.5 mM were added) and incubated for one hour at 37 °C under stirring (180 RPM). After transformation, the bacterial strains were propagated in LB solid medium, to which suitable antibiotics for selection of the transformed bacteria were added, and incubated at 37 °C overnight. After 24 h, single colonies of transformed bacteria were inoculated in 2-3.5 mL of LB liquid medium, to which suitable antibiotics were added, and grown under constant stirring at 37 °C, for 15 hours (preculture). For expression of the recombinant proteins, the preculture was diluted 1:500 and inoculated in the same medium (culture). The cultures were first grown under constant stirring (180 RPM), at 30°C for 9 hours, then the expression of the recombinant protein was induced where necessary with IPTG (isopropyl β-D-1-thiogalactopyranoside 1 mM) for 15h. Aliquots of 60 mL of culture of transformed bacteria containing the recombinant proteins were centrifuged for 40 min at 4400 RPM at 4 °C and the cell pellets obtained were stored at -20 °C until their use.

### COMPARATIVE EXAMPLE 1: sonication

A cell pellet was obtained from *E. coli* BL21(DE3) (Novagen^{®}) transformed as in Example 1 with the plasmid pETDuet-1, comprising gene 2, as described above. The resulting plasmid is indicated as pETDuet-Gene2. From this pellet of these bacteria, enzyme 2 was extracted by sonication: briefly, the transformed cell pellets were resuspended in 40 mL of a lysis solution (Tris HCl pH 8, NaCl 50 mM, EDTA 1 mM) and subjected to three or four sonication cycles at 1.5 W. The sonicator utilised is a Misonix Sonicator 3000 (Qsonica, CT, USA) .

Solubility of the extracted enzyme was evaluated by SDS-PAGE analysis. In the present and in the subsequent examples, a molecular weight marker (M) obtained from Fermentas was used; from top to bottom the molecular weight bands are: 116.0 kDa, 66.2 kDa, 45.0 kDa, 35.0 kDa, 25.0 kDa, 18.4 kDa. Comparing in Fig. 1 the total cellular extract (lane 1) with the soluble extract (lane 2) obtained as supernatant by centrifuging the solution obtained after sonication at 13000 RPM for 20 minutes at 4°C, it can be noted that the enzyme 2 extracted is mainly soluble.

### EXAMPLE 2: Extraction at pH 13 and 45 °C

A pellet of *E. coli* BL21(DE3) cells transformed as in the Comparative Example 1 was resuspended in phosphate buffer 100 mM at pH 8; the same volume of NaOH 0.2 M was then added. The reaction mixture was incubated for different times (0, 10, 30, 60, 120, 180, 270, 360 min) at the temperature of 45 °C; the pH of the incubation mixture under stirring was measured as 13. At the end of each incubation time, aliquots of 1 mL were collected from the mixture obtained. Each aliquot was subjected to centrifugation at 13000 RPM, for 20 min at 4 °C: each supernatant (soluble extract, containing the extracted proteins) was analysed by electrophoresis on polyacrylamide gel (SDS-PAGE).

It was found that extraction of the enzyme in these conditions occurs instantly already after a few seconds (lane 2, Fig. 2); the largest amount of soluble enzyme 2 is obtained by extracting the sample for 10-60 minutes (Fig. 2, lanes 3 to 5). The amount of enzyme obtained in just 10 minutes of extraction is comparable to, if not higher than, the amount found in the soluble extract (lane 1, Fig. 2) obtained by sonication as in Comparative Example 1, which forms the reference control.

### COMPARATIVE EXAMPLE 2: extraction at pH 9-10

A pellet of *E. coli* BL21 (DE3) cells transformed as in the Comparative Example 1 was resuspended in phosphate buffer at pH 8; suitable volumes of a solution of NaOH 0.2 M were then added, setting up extraction reactions in an incubation mixture having a pH of from 9 to 10. The incubations were carried out at 45 °C at different times (0-6h). No effective extractions were obtained in any of these conditions (Fig. 11).

### COMPARATIVE EXAMPLE 3: extraction at pH 10-12

A pellet of *E. coli* BL21 (DE3) cells transformed as in Comparative Example 1 was resuspended in phosphate buffer 100 mM at pH 8; suitable volumes of a solution of NaOH 0.2 M were then added, setting up extractions in incubation mixtures having pH 10, 11 or 12, at 56 °C for a time of 60 minutes. By analysing the soluble extracts by SDS-PAGE (Fig. 12) it can be observed that none of the conditions examined are optimal for extracting enzyme 2 from the cells. The reaction carried out at pH 12 resulted in a slight extraction of enzyme 2 (lanes 5 and 8).

### COMPARATIVE EXAMPLE 4: extraction with detergents

A detergent (Sodium dodecyl sulphate, SDS, 1% w/v or hexadecyl trimethyl ammonium bromide, CTAB, 5% w/v) was added to pellets of E. coli BL21 (DE3) cells transformed as in Comparative Example 1, resuspended in phosphate buffer 100 mM at pH 8 and incubated at different temperatures and extraction times, as indicated in Table 1.

**Table 1**

| Reaction | SDS | CTAB | pH | Temperature (°C) | Time (min) |
|---|---|---|---|---|---|
| 1 | 1 *‰* | / | 12 | 56 | 60 |
| 2 | 1 *‰* | / | 11 | 56 | 60 |
| 3 | 1 *‰* | / | 10 | 56 | 60 |
| 4 | 1 *‰* | / | 9 | 56 | 60 |
| 5 | 1 *%* | / | 9 | 56 | 60 |
| 6 | 1 *‰* | / | 8 | 56 | 60 |
| 7 | 1 *%* | / | 8 | 56 | 60 |
| 8 | / | 1 *‰* | 8 | 56 | 60 |
| 9 | / | 1 *%* | 8 | 56 | 60 |
| 10 | / | 1 *‰* | 9 | 56 | 60 |
| 11 | / | 1 *%* | 9 | 56 | 60 |
| 12 | 1 *%* | / | 8 | 100 | 5 |
| 13 | 1 *%* | / | 8 | 100 | 10 |
| 14 | 1 *%* | / | 8 | 100 | 15 |
| 15 | 1 *%* | / | 8 | 100 | 20 |
| 16 | 1 *%* | / | 8 | 100 | 30 |
| 17 | 1 *%* | / | 8 | 100 | 60 |

The soluble extracts obtained from each reaction were analysed by SDS-PAGE (Figs. 13, 14, 15).

### EXAMPLE 3: extraction at room temperature

Suitable volumes of a solution of NaOH 0.2 M were added to a pellet of transformed *E. coli* BL21(DE3) cells, resuspended in phosphate buffer as in Comparative Example 1, so as to obtain an incubation mixture having a pH of 10, 11, 12 and 13. Each extraction was carried out for 15 hours at 20 °C.

The SDS-PAGE analysis, shown in Fig. 3, indicates that it is possible to effectively extract recombinant proteins incubating a pellet of bacteria in the presence of an incubation mixture with a pH of 13 (lane 5), also at room temperature. The reference control is formed by the soluble cellular extract obtained by sonication (lane 1).

### EXAMPLE 4: extraction at pH 13 at room temperature

An incubation mixture having a pH of 13 was set up as in example 3. Aliquots of the total extracts were collected after 3, 6, 9 and 15 hours from the start of the extraction incubation. A suitable volume of an acid solution (HCl 0.2 M) was added to each aliquot to neutralise the basic incubation mixture, returning the pH of the suspension to a value of 8. Subsequently, the soluble protein extracts were isolated by centrifugation and analysed by SDS-PAGE (Fig. 4). Analysis showed that an extraction of 3 h at 25°C (lane 2 in Fig. 4) is sufficient to extract the recombinant protein of interest. The amount of enzyme extracted progressively increases after 6 and 9 h of extraction (lanes 3 and 4 in Fig.4). Extension of the extraction time to 15 h (lane 5 in Fig. 4) determines a slight decrease of the yield.

### EXAMPLE 5: assay of protein extract activity

The protein extracts obtained in Example 4, after 3, 6 and 15 h of extraction, were utilised to assay β-glucosidase activity, in which the capacity of the extracted enzyme to catalyse hydrolysis of the substrate p-nitrophenyl-β-d-glucopyranoside (pNPGluc) in p-nitrophenolate and glucose was measured. pNPGluc 1 mM in solution Tris-HCl 50 mM, KCl 50 mM, pH 8 was utilised for the assays at the temperature of 25 °C, pNPGluc 1 mM in a citrate buffer solution 50 mM at pH 5 for the assays at the temperature of 40, 60 or 80 °C. The enzymatic reaction was monitored by a UV/VIS spectrophotometer (Cary 300): the variation of absorbance at 420 nm as a function of time was detected for 20 minutes and linearly interpolated (zero order), thus obtaining a slope value expressed in ΔAbs/min. Each value was subsequently converted utilising the molar extinction coefficient of the reaction product (ε = 12900 M⁻¹ cm⁻¹) .

β-glucosidase activity was detected in each of the extracts, proving that enzyme 2 extracted at pH 13, at 20 °C, even in just 3 h, is biologically active.

### EXAMPLE 6: termination of the extraction

Enzyme 2 was extracted at 25 °C and pH 13 from a cell pellet of *E. coli* BL21(DE3), as described in Example 4, for variable times (from 1 to 6 hours). Aliquots of incubation mixture were collected every hour after the start of the reaction and a suitable amount of HCl 0.2 M was added to return the solution to a pH of 8.

SDS-PAGE analysis of the soluble extracts (E Sol) shows that the amount of extracted enzyme 2 progressively increases from 1 to 6 hours of extraction (lanes 2 to 7 in Fig. 5). At an extraction prolonged for 4-5 h, the amount of extracted enzyme 2 is comparable to the amount obtained by sonication (E Sol Sonicator, reference control). However, the extraction carried out with sonicator is less selective with respect to the extraction according to the method of the invention, as significant amounts of other soluble proteins are also extracted.

The concentration of proteins of each soluble extract (mgP/mL) was measured by a Bradford assay. The results are set down in Table 2.

**Table 2**

| Sample soluble extract | mgP/mL |
|---|---|
| Sonicator (Control) | 0.164 |
| pH 13 for 1 hour | 0.085 |
| pH 13 for 2 hours | 0.100 |
| pH 13 for 3 hours | 0.116 |
| pH 13 for 4 hours | 0.122 |
| pH 13 for 5 hours | 0.139 |
| pH 13 for 6 hours | 0.140 |

### EXAMPLE 7: Study of the β-glucosidase activity

The soluble extracts of Example 6 were utilised to carry out assays of β-Glucosidase activity at 25 °C, as described in Example 5. The analysis showed that the β-glucosidase activity detected after extraction with sonication (control, Fig. 6a) is lower than that detected for the samples extracted according to the method of the present invention; maximum activity is detected in the soluble extract obtained after 2 h of extraction at pH 13 (2h, Fig. 6a).

By calculating the specific activities of each soluble extract it was found that the highest value is the value of the sample obtained after 2 h of extraction at pH 13 (Fig. 7, while balls) . In fact, with respect to sonication, with the same mg of protein, the catalytic activity of proteins obtained according to the method of the invention in 2 hours of incubation is higher (more than double). It is thought that a part of the protein concentration detected with the Bradford assay in extracts obtained by sonication could be due to the presence of proteins other than enzyme 2. Moreover, by developing heat, the sonication method could denature part of enzyme 2.

### EXAMPLE 8: simultaneous extraction of several proteins

In the pETDuet-1 plasmid, gene 1 as described above and gene 2 as described above were respectively cloned in a first multiple cloning site and in a second site; the resulting plasmid is indicated as pETDuet-Gene1-Gene2. In the pACYCDuet-1 plasmid, the gene 3 as described above was cloned; the resulting plasmid is indicated as pACYCDuet-Gene 3. Cells of *E. coli* BL21(DE3) were transformed with the vector pETDuet-Gene1-Gene2 or with both the vectors pETDuet-Gene1-Gene2/pACYCDuet-Gene3 and the respective pellets were utilised to extract, respectively, enzymes 1 and 2 (G1+G2) or enzymes 1, 2 and 3 (G1+G2+G3) by sonication or by a method according to the present invention.

Comparing by SDS-PAGE analysis (Fig. 8) the total cellular extract (lanes 1 and 3) and the soluble extract (lanes 2 and 4) obtained by sonication, it can be noted that the enzymes so extracted are mostly insoluble, probably due to their significant denaturation.

SDS-PAGE analysis of total (lanes 3 and 4) and soluble (lanes 1 and 2) protein extracts obtained by incubation of pellets of bacteria transformed with one (Fig. 9 b) or two vectors (Fig. 9 a) at room temperature and at a pH value of 13, terminated after 2 or 2.5 hours adding HCl 0.2 M, so as to interrupt extraction, returning the reaction solution to a pH value of 8, shows that the enzymes extracted according to a preferred method of the present invention (E1, E2, E3) are mostly soluble.

Therefore, the extraction procedure of the present invention has proved to be more effective than sonication, to extract enzymes, even simultaneously, maintaining them in soluble form.

### EXAMPLE 9: quantification of the proteins in soluble extracts and analysis of activity

The soluble extracts obtained in Example 8 by extraction at pH 13 at 25°C for 2 hours were utilised to quantify the extracted proteins by a Micro-Bradford assay as described above. The results are set down in Table 3 as mg of proteins found in 1 mL (mgP/mL) of each soluble extract.

**Table 3**

| Enzymes extracted | mgP/mL |
|---|---|
| Enzymes 1, 2 and 3 | 0.174 |
| Enzymes 1 and 2 | 0.136 |

Aliquots of the same extracts were utilised to carry out assays of β-glucosidase activity.

pNPGluc at a final concentration of 1 mM was administered as substrate of each reaction. The control sample (white) has no enzymes. The reaction was set up at 80°C and the progressive formation of p-nitrophenolate was examined after 5, 10, 15, 20, 30, 40, 50 and 60 minutes from the start of the reaction.

The reaction set up using 10 µL of soluble extract (0.27 ug/mL of proteins) from bacteria containing enzyme 1 and 2 had a velocity of 4.26 µM/min (Fig. 10a). The corresponding reaction performed with 10 µL of soluble extract (0.35 ug/mL of proteins) from bacteria containing enzyme 1, 2 and 3 had a reaction velocity of 4.6 µM/min (Fig. 10, b). The amount of substrate that was hydrolysed in only 60 min in the reaction at 80 °C corresponds to approximately 50% of the total initial substrate (initial pNPGluc = 1mM).

### EXAMPLE 10: Cellulose activity assays

Soluble protein extracts of Example 8 were also utilised to verify their capacity to digest sodium carboxymethylcellulose (CMC) and microcrystalline cellulose. Each cellulose substrate was utilised at the final concentration of 10 mg/mL. 1 mL of soluble extract containing enzymes 1 and 2 (G1&G2) or enzymes 1, 2 and 3 (G1&G2&G3) was used for each assay. For the assays at 80°C (at pH 5) a citrate buffer solution 50 mM, pH 4 was utilised, while for the assays at 100 °C (at pH 6) a citrate buffer solution 50 mM, pH 5 was utilised. For the blank assays suitable amounts of phosphate buffer 100 mM, EDTA 5 mM, pH 8 were used in substitution of the soluble protein extract. The glucose present in the samples was analysed by a glucometer (Roche), normalised with respect to standard samples (calibration curve y=7.6915x-14.237).

For the activity assays at a temperature of 80°C at 10 mg/mL of CMC or of microcrystalline cellulose, were placed suitable amounts of citrate buffer 50 mM, pH 4 were added. 200 µE/mL of soluble protein extract were then added to each reaction solution. Some controls (white) were left at room temperature (25 °C) for the whole of the duration of the experiment. Aliquots of 200 µL were collected at different times and the concentration of glucose determined.

Table 4 summarises the samples analysed.

**Table 4**

| Reaction | Sample | Microcrystalline cellulose (mg/mL) | CMC (mg/mL) | Temperature (°C) |
|---|---|---|---|---|
| 1 | G1&G2&G3 | 10 | / | 80 |
| 2 | G1&G2 | 10 | / | 80 |
| 3 | White 80 °C | 10 | / | 80 |
| 4 | White room t | 10 | / | 80 |
| 5 | G1&G2&G3 | / | 10 | 80 |
| 6 | G1&G2 | / | 10 | 80 |
| 7 | White 80 °C | / | 10 | 80 |
| 8 | White room t | / | 10 | 80 |

After 18, 26 and 42 hours (h) from the start of incubation, each solution was subjected to centrifugation and the concentration of glucose released by the enzyme action was evaluated in the supernatants (Table 5). The pellets obtained were washed twice with 5 mL of H₂O, then dried in an oven at 105 °C for 9.5 h and the dry weight was calculated.

**Table 5**

| Reaction | Sample | Glucose 18 h (g/L) | Glucose 26 h (g/L) | Glucose 42 h (g/L) |
|---|---|---|---|---|
| 1 | G1&G2&G3 | 0 | 0 | 0 |
| 2 | G1&G2 | 0 | 0 | 0 |
| 3 | White | 0 | 0 | 0 |
| 4 | White room t | 0 | 0 | 0 |
| 5 | G1&G2&G3 | 0.9 | 0.9 | 0.9 |
| 6 | G1&G2 | 0.92 | 0.9 | 0.92 |
| 7 | White 80 °C | 0 | 0 | 0 |
| 8 | White room t | 0 | 0 | 0 |

Some samples (G1&G2&G3-CMC-42h, G1&G2-CMC-42h and White 80 °C-CMC-42h) were also analysed by HPLC (see Table 6 for the results).

**Table 6**

| Reaction | Sample | CMC (g/L) | Glucose 42 h(g/L) |
|---|---|---|---|
| 5 | G1&G2&G3 | 0.13 | 1.18 |
| 6 | G1&G2-CMC- 42h | 0.11 | 1.39 |
| 7 | White 80°-CMC-42h | 2.71 | 0 |

In 42 hours (42h) 90% of the CMC substrate was converted into glucose by the extracted enzymes.

### EXAMPLE 11: extraction of further proteins

The method of the present invention was utilised for the extraction at pH 13, from transformed *Escherichia coli* bacteria, of the following recombinant proteins: tyrosine phosphatase (TP) from *Mycobacterium tuberculosis* (sequence SEQ ID NO. 6) and synthetic Klenow sub-fragment (HoLaMa) from *Escherichia coli* (sequence SEQ ID NO. 7). The bacteria were transformed with a vector having the nucleotide sequence SEQ ID NO. 8, encoding for tyrosine phosphatase or with a vector having the nucleotide sequence SEQ ID NO. 9 encoding for the synthetic Klenow sub-fragment (HoLaMa); the transformed bacteria were resuspended in phosphate buffer 100mM at pH8, supplemented with 5mM EDTA for extraction of tyrosine phosphatase, while for extraction of the Klenow sub-fragment beta-mercaptoethanol 10mM was added to the phosphate buffer; a volume of basic solution suitable to obtain an incubation mixture at pH 13 was then added to the bacterial suspension. The mixture comprising the extracted proteins was then analysed by SDS-PAGE in acrylamide/bisacrylamide gel at 15%, comparing it with an extraction mixture obtained by sonication (Fig. 14: tyrosine phosphatase from *Mycobacterium tuberculosis, 18.0 kDa);* Fig. 15: synthetic Klenow sub-fragment from *Escherichia coli,* 46.2 kDa).

### EXAMPLE 12: analysis of activity of the extracted proteins

The activity of proteins extracted in the previous example with half an hour of incubation at pH 13 was assayed: for tyrosine phosphatase by determining the increase in similarity to 405 nm, indicative of hydrolysis of the p-nitrophenylphosphate substrate; for the Klenow sub-fragment (HoLaMa) as described in Martina et al. 2015 ¹³, by using the substrate of DNA 40mer indicated in the same paper.

The results are shown in Tables 7 (for tyrosine phosphatase) and 8 (Klenow sub-fragment).

**Table 7**

| Extraction | Activity | Protein concentration | Specific |
|---|---|---|---|
| | (nM/sec) | (mg/mL) | Activity (µM/sec*mg P) |
| Sonication | 972 | 0.73 | 13.3 |
| Basic solution 0.5 hours | 978 | 0.12 | 81.5 |

**Table 8**

| Extraction | Activity (nM/sec) | Protein concentration (mg/mL) | Specific Activity (µM/sec*mg P) |
|---|---|---|---|
| Sonication | 0.77 | 0.39 | 19,7 |
| Basic solution 0.5 hours | 0.99 | 0.39 | 25,4 |

The method of the present invention has shown to be more effective with respect to conventional extraction methods; it is in fact more selective, allowing extraction of the recombinant proteins of interest, to the detriment of the bacterial proteins, having a greater yield of proteins, in particular of soluble proteins, preserving their biological activity to a greater extent and being also more economical.

### Bibliography

1. Kengen, Serve WM, et al. "Purification and characterization of an extremely thermostable β-glucosidase from the hyperthermophilic archaeon Pyrococcus furiosus." European Journal of Biochemistry 213.1 (1993): 305-312.
2. Voorhorst, W. G., et al. "Characterization of the CelB gene coding for beta-glucosidase from the hyperthermophilic archaeon Pyrococcus furiosus and its expression and site-directed mutation in Escherichia coli." Journal of bacteriology 177.24 (1995): 7105-7111.
3. Zhang, Sheng, Diana C. Irwin, and David B. Wilson. "Site-directed mutation of noncatalytic residues of Thermobifida fusca exocellulase Cel6B." European Journal of Biochemistry 267.11 (2000): 3101-3115.
4. Gomez del Pulgar, Eva Maria, and Anas Saadeddin. "The cellulolytic system of Thermobifida fusca." Critical reviews in microbiology 40.3 (2014): 236-247.
5. Zhang, Sheng, Guifang Lao, and David B. Wilson. "Characterization of a Thermomonospora fusca exocellulase." Biochemistry 34.10 (1995): 3386-3395.
6. F.W. Studier, B.A. Moffatt, Use of bacteriophage T7 RNA polymerase to direct selective high-level expression of cloned genes, J. Mol. Biol. 189 (1986) 113-130.
7. Chamberlin, Michael, Janet McGrath, and Lucy Waskell, New RNA polymerase from Escherichia coli infected with bacteriophage T7. Nature 228 (1970): 227-231.
8. J.W. Dubendorff, F.W. Studier, Controlling basal expression in an inducible T7 expression system by blocking the target T7 promoter with lac repressor, J. Mol. Biol. 219 (1991) 45-59.
9. Bhalla, Aditya, et al. "Improved lignocellulose conversion to biofuels with thermophilic bacteria and thermostable enzymes." Bioresource technology 128 (2013): 751-759.
10. Watson et al., DNA ricombinante, 2008, Zanichelli.
11. Wood, W.B. 1966. Host specificity of DNA produced by Escherichia coli: bacterial mutations affecting the restriction and modification of DNA. J Mol Biol. 16:118-133
12. Phillips T.A. et al. Ion gene product of Escherichia coli is a heat-shock protein. J Bacteriol 159, 283-287.
13. Martina et al., 2015, Archives of Biochemistry and Biophysics, 575: 46-53.

## Claims

1. A method for extracting recombinant proteins from bacteria, comprising the steps of:
i. adding a basic solution to a suspension of bacteria containing said recombinant proteins, thus obtaining an incubation mixture having a pH of 12 or more;
ii. incubating said bacteria in said incubation mixture having a pH in the range of 12 and 14;
iii. at the end of the incubation, adding a volume of acid solution to the incubation mixture, such as to obtain a final mixture having a pH ranging between 7 and 9;
iv. purifying the recombinant proteins extracted from the incubation mixture or from the final mixture;
Wherein:
- said basic solution is a solution of sodium hydroxide (NaOH);
- said bacteria are incubated in said incubation mixture for a time ranging between 1 and 3 hours;
- said bacteria are incubated in said incubation mixture at a temperature ranging between 18°C and 30°C;
and wherein said bacteria are gram negative.

2. A method according to claim 1 wherein said bacteria are *Escherichia coli* , preferably of the BL21(DE3) strain.

3. A method according to any one of the preceding claims, wherein said bacteria are bacteria transformed with one or more expression vectors comprising at least one polynucleotide encoding for said recombinant proteins.

4. A method according to any one of the preceding claims, wherein said incubation mixture has a pH of 13.

5. A method according to any one of the preceding claims, wherein said bacteria are incubated in said incubation mixture at room temperature.

6. A method according to any one of the preceding claims, wherein said recombinant proteins are thermostable proteins.

7. A method according to any one of the preceding claims, wherein said recombinant proteins are cellulases.

8. A method according to any one of the preceding claims, wherein said extracted recombinant proteins are purified by flocculation or by centrifugation of the incubation mixture or from the final mixture.

9. Use of NaOH for the extraction of recombinant protein from bacteria according to the method of any one of claims 1 to 10.

## Patentansprüche

1. Verfahren zum Extrahieren von rekombinanten Proteinen aus Bakterien, umfassend die folgenden Schritte:
i. Hinzufügen einer basischen Lösung zu einer Suspension von Bakterien, die die rekombinanten Proteine enthalten, wodurch ein Inkubationsgemisch erlangt wird, das einen pH von 12 oder mehr aufweist;
ii. Inkubieren der Bakterien in dem Inkubationsgemisch, das einen pH in dem Bereich von 12 und 14 aufweist;
iii. am Ende der Inkubation, Hinzufügen eines Volumens saurer Lösung zu dem Inkubationsgemisch, um ein abschließendes Gemisch zu erlangen, das einen pH zwischen 7 und 9 aufweist;
iv. Aufreinigen der rekombinanten Proteine, die aus dem Inkubationsgemisch oder dem abschließenden Gemisch extrahiert werden;
wobei:
- die basische Lösung eine Lösung von Natriumhydroxid (NaOH) ist;
- die Bakterien über eine Zeit zwischen 1 und 3 Stunden in dem Inkubationsgemisch inkubiert werden;
- die Bakterien bei einer Temperatur zwischen 18 °C und 30 °C in dem Inkubationsgemisch inkubiert werden;
und wobei die Bakterien gramnegativ sind.

2. Verfahren nach Anspruch 1, wobei die Bakterien *Escherichia coli,* vorzugsweise vom Stamm BL21(DE3), sind.

3. Verfahren nach einem der vorherigen Ansprüche, wobei die Bakterien Bakterien sind, die mit einem oder mehreren Expressionsvektoren transformiert sind, umfassend mindestens ein Polynukleotid, das für die rekombinanten Proteine kodiert.

4. Verfahren nach einem der vorherigen Ansprüche, wobei das Inkubationsgemisch einen pH von 13 aufweist.

5. Verfahren nach einem der vorherigen Ansprüche, wobei die Bakterien bei Raumtemperatur in dem Inkubationsgemisch inkubiert werden.

6. Verfahren nach einem der vorherigen Ansprüche, wobei die rekombinanten Proteine thermostabile Proteine sind.

7. Verfahren nach einem der vorherigen Ansprüche, wobei die rekombinanten Proteine Cellulasen sind.

8. Verfahren nach einem der vorherigen Ansprüche, wobei die extrahierten rekombinanten Proteine durch Ausflockung oder Zentrifugation des Inkubationsgemischs oder aus dem abschließenden Gemisch aufgereinigt werden.

9. Verwendung von NaOH für die Extraktion von rekombinantem Protein aus Bakterien gemäß dem Verfahren nach einem der Ansprüche 1 bis 10.

## Revendications

1. Procédé d'extraction de protéines de recombinantes à partir de bactéries, comprenant les étapes suivantes :
i) l'ajout d'une solution basique à une suspension de bactéries contenant lesdites protéines recombinantes, obtenant ainsi un mélange d'incubation ayant un pH supérieur ou égal à 12 ;
ii) l'incubation desdites bactéries dans le mélange d'incubation dont le pH est compris entre 12 et 14 ;
iii) à la fin de l'incubation, l'ajout d'un volume de solution acide au mélange d'incubation, de manière à obtenir un mélange final dont le pH est compris entre 7 et 9 ;
iv) la purification des protéines recombinantes extraites du mélange d'incubation ou du mélange final ;
dans lequel,
- ladite solution basique est une solution d'hydroxyde de sodium (NaOH) ;
- lesdites bactéries sont incubées dans ledit mélange d'incubation pendant une durée comprise entre 1 et 3 heures ;
- lesdites bactéries sont incubées dans ledit mélange d'incubation à une température comprise entre 18°C et 30°C ;
et dans laquelle lesdites bactéries sont gram négatives.

2. Procédé selon la revendication 1, dans lequel lesdites bactéries sont de la souche *Escherichia coli,* de préférence de la souche BL21(DE3).

3. Procédé selon l'une quelconque des revendications précédentes, dans lequel lesdites bactéries sont des bactéries transformées avec un ou plusieurs vecteurs d'expression comprenant au moins un polynucléotide codant pour lesdites protéines recombinantes.

4. Procédé selon l'une quelconque des revendications précédentes, dans lequel ledit mélange d'incubation a un pH de 13.

5. Procédé selon l'une quelconque des revendications précédentes, dans lequel lesdites bactéries sont incubées dans ledit mélange d'incubation à température ambiante.

6. Procédé selon l'une quelconque des revendications précédentes, dans lequel lesdites protéines recombinantes sont des protéines thermostables.

7. Procédé selon l'une quelconque des revendications précédentes, dans lequel lesdites protéines recombinantes sont des cellulases.

8. Procédé selon l'une quelconque des revendications précédentes, dans lequel lesdites protéines recombinantes extraites sont purifiées par floculation ou par centrifugation du mélange d'incubation ou du mélange final.

9. Utilisation de NaOH pour l'extraction de protéines recombinantes de bactéries suivant le procédé selon l'une quelconque des revendications 1 à 10.
